# EUROPEAN PATENT SPECIFICATION

(11) **EP 3 151 800 B1**
(45) Date of publication and mention of the grant of the patent: **13.11.2019**
(21) Application number: 15730022.9
(22) Date of filing: 05.06.2015
(51) Int. Cl.: A61F 13/476, A61F 13/56, A61F 13/551

(54) **DISPOSABLE ABSORBENT ARTICLE HAVING UNDERGARMENT FASTENING ELEMENTS**
SAUGFÄHIGER EINWEGARTIKEL MIT BEFESTIGUNGSELEMENTEN FÜR UNTERWÄSCHE
ARTICLE ABSORBANT JETABLE AVEC ÉLÉMENTS DE FIXATION POUR SOUS-VÊTEMENT

(30) Priority: 06.06.2014 US 201414298122
(43) Date of publication of application: 12.04.2017
(73) Proprietor: The Procter & Gamble Company, Cincinnati, OH 45202 (US)
(72) Inventor: LANGDON, Frederick Michael, Cincinnati, Ohio 45224 (US)
(74) Representative: P&G Patent Germany
(86) International application number: PCT/US2015/034428
(87) International publication number: WO 2015/188074

(56) References cited:
- EP-A2- 0 852 133
- WO-A1-93/20790
- WO-A1-96/23469
- WO-A1-97/47266
- WO-A1-98/27903
- US-A- 5 662 639
- US-A1- 2005 124 958

## Description

### FIELD OF THE INVENTION

This invention relates to absorbent articles such as catamenial devices and light incontinence products. In particular, this invention relates to catamenial devices such as sanitary napkins having improved comfort and body fit.

### BACKGROUND OF THE INVENTION

Sanitary napkins and related disposable absorbent articles that provide for the collection of menses and other bodily discharges are well known in the art. Some commercially available sanitary napkins have pressure sensitive adhesive on their backsheets for affixing the sanitary napkin to the undergarment of the wearer which can help maintain the sanitary napkin in the proper position to intercept the discharged bodily fluid. Additionally, some commercially available sanitary napkins include side flaps which can adhere to the undergarments of the wearer via adhesive and further help to maintain the sanitary napkin in the proper position.

US 2005/124958, June 9^{th} 2005, discloses a disposable sanitary napkin with a main body and a pair of discrete flaps. Each flap includes an attachment portion secured to the main body.

WO 93/20790, October 28^{th} 1993, discloses a sanitary napkin comprising side panels which are folded over a top sheet such that adhesive segments on the side panels are arranged side by side, the side panels being held in position by a panel securing strip bonded to the first and second adhesive segments.

US 5,662,639, issued on September 2^{nd} 1997, discloses a sanitary napkin having a pair of wings extending outward from transversely opposite side edges of the napkin. The wings are folded back onto a top surface of the napkin and provided on lower surfaces of the wings with first and second adhesive zones, respectively.

Typically, such sanitary napkins are provided with two separate release liners - one covering the pressure sensitive adhesive of the backsheet and the other covering the pressure sensitive adhesive of the side flaps. Typically, the user removes the first release liner from the backsheet and attaches the sanitary napkin to the undergarment. Subsequently, the user removes the second release liner from the side flaps and then folds the side flaps over the undergarment thereby sandwiching the undergarment between the side flaps and the backsheet. Because the pressure sensitive adhesive of the backsheet and the side flaps can readily adhere to many different surfaces, the attachment of the sanitary napkin to an undergarment is often times not a trivial exercise.

Additionally, side flaps are often subjected to stresses during use. For example, the movement of the wearer, e.g. standing, sitting, running, etc., can cause the pressure sensitive adhesive to release from the undergarment and cause discomfort for the wearer. Unfortunately, often times the design of the sanitary napkin further contributes to the stresses applied to the side flaps during use further compromising the fit / comfort of the sanitary napkin.

Accordingly, there is a need for a sanitary napkin having side flaps configured to facilitate the adherence of the sanitary napkin to the undergarment of the user. Additionally, there is a need for a sanitary napkin having side flaps which improves the fit / comfort to the wearer while reducing stresses on the side flaps during use.

### SUMMARY OF THE INVENTION

Absorbent articles constructed in accordance with the present invention can facilitate the application of the absorbent article to an undergarment of a user as described herein. The present invention relates to an absorbent article having a longitudinal axis, a lateral axis extending generally perpendicular to the longitudinal axis, and a vertical axis extending generally perpendicular to the longitudinal axis and the lateral axis, the absorbent article further comprising:
a chassis comprising a topsheet, a backsheet, and an absorbent core disposed between the topsheet and the backsheet, the chassis having a body-facing surface, an outer-facing surface on the opposite surface facing away from the wearer's body during use, and the chassis being defined by a periphery comprising first and second longitudinal edges;
a pair of discrete side flaps each having a first surface and a second surface opposite the first surface and each further comprising attached areas and free ends, wherein the attached area of each side flap is attached to the chassis such that a portion of the first surface of each side flap is attached to the outer-facing surface of the chassis, wherein an adhesive is disposed on a portion of the first surface of each of the side flaps adjacent the free end; and
a release liner covering at least a portion of the adhesive on the first surface of each of the side flaps, the release liner and the free ends of each of the side flaps being disposed vertically superjacent to the chassis when the pair of discrete side flaps is in an unfolded state and oriented where the topsheet is positioned superjacent to the absorbent core.

### BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings, in which:
Figure 1 is a plan view depicting an absorbent article constructed in accordance with the present invention in a partially unfolded state;
Figure 2A is a modified cross sectional view depicting side flaps of the absorbent article of Figure 1 hanging downward;
Figure 2B is a close up view depicting a flange seam of the absorbent article of Figure 1;
Figure 3 is a cross sectional view depicting the absorbent article of Figure 1 in a partially unfolded state with a release liner in place;
Figure 4 is a close up view depicting another embodiment for a flange seam for use with absorbent articles of the present invention;
Figure 5 is a close up view depicting another embodiment for a flange seam for use with absorbent articles of the present invention;
Figure 6 is a close up view depicting another embodiment for a flange seam for use with absorbent articles of the present invention;
Figure 7 is a close up view depicting another embodiment for a flange seam for use with absorbent articles of the present invention;
Figure 8 is a close up view depicting another embodiment for a flange seam for use with absorbent articles of the present invention;
Figure 9 is a close up view depicting another embodiment for a flange seam for use with absorbent articles of the present invention; and
Figure 10 is a plan view depicting an absorbent article constructed in accordance with the present invention in a partially unfolded state having a plurality of pairs of side flaps.

### DETAILED DESCRIPTION OF THE INVENTION

The present invention may be utilized in a sanitary napkin and many different absorbent articles as provided below. As used herein, the phrase "absorbent article" refers to devices which absorb and contain body liquids, and more specifically refers to devices which may be placed against or near the skin to absorb and contain the various liquids, such as those discharged from the body. In typical use the absorbent articles are not intended to be laundered or otherwise restored or reused after a single use. Examples of absorbent articles include, but are not limited to: personal care absorbent products, such as: feminine hygiene products, for example feminine sanitary napkins, pantiliners, tampons, interlabial devices and the like and adult incontinence products.

Absorbent articles, and their individual components, such as a liquid pervious topsheet, a substantially liquid impervious backsheet joined to the topsheet, and an absorbent core positioned and held between the topsheet and the backsheet, have a body-facing surface and an outer-facing surface. As used herein, "body-facing surface" means that surface of the article or component which is intended to be disposed toward or placed adjacent to the body of the wearer during ordinary use, while the "outer-facing surface" is on the opposite side, and is intended to be disposed to face away from the wearer's body during ordinary use. The outer-facing surface may be arranged to face toward or placed adjacent to the wearer's undergarments or outer garments when the absorbent article is worn.

The term "nonwoven" or "nonwoven material" refers herein to a material made from continuous (long) filaments (fibers) and/or discontinuous (short) filaments (fibers) by processes such as spunbonding, meltblowing, carding, and the like. Nonwovens do not have a woven or knitted filament pattern.

As used herein, the term "nonwoven web" means a manufactured sheet, web, or batt of directionally or randomly orientated fibers, bonded by friction, and/or cohesion, and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers may have diameters ranging from less than about 0.001 mm to more than about 0.2 mm and may come in several different forms such as short fibers (known as staple, or chopped), continuous single fibers (filaments or monofilaments), untwisted bundles of continuous filaments (tow), and twisted bundles of continuous filaments (yam). Nonwoven webs may be formed by many processes such as meltblowing, spunbonding, solvent spinning, electrospinning, carding, and airlaying. The basis weight of nonwoven webs is usually expressed in grams per square meter (g/m2 or gsm).

As used herein, the terms "joined", "bonded", or "attached" encompasses configurations whereby an element is directly secured to another element by affixing the element directly to the other element, and configurations whereby an element is indirectly secured to another element by affixing the element to intermediate member(s) which in turn are affixed to the other element.

As used herein, the term "hydrophilic", refers to a material having a contact angle less than or equal to 90° according to The American Chemical Society Publication "Contact Angle, Wettability, and Adhesion," edited by Robert F. Gould and copyrighted in 1964.

As used herein, the term "hydrophobic", refers to a material or layer having a contact angle greater than or equal to 90° according to The American Chemical Society Publication "Contact Angle, Wettability, and Adhesion," edited by Robert F. Gould and copyrighted in 1964.

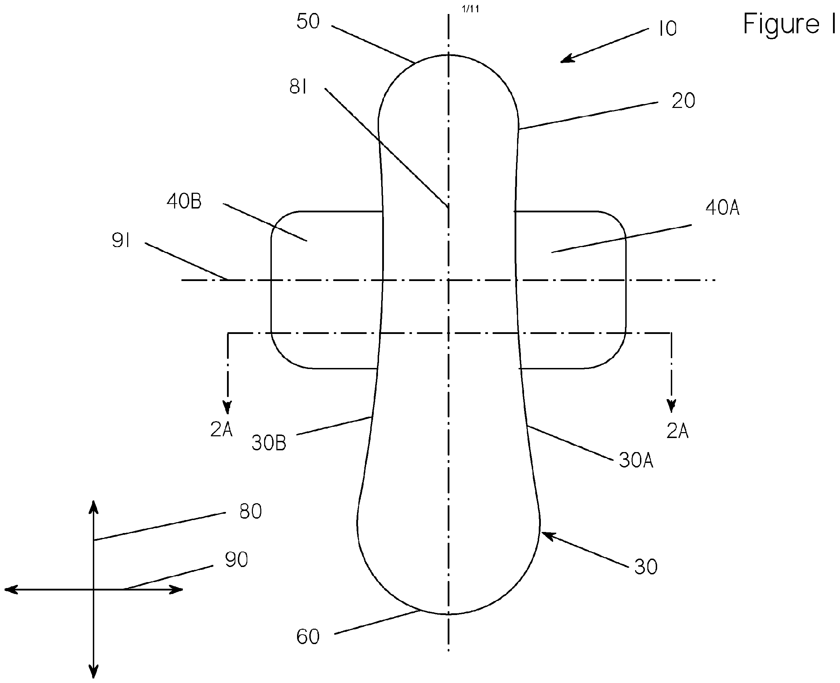

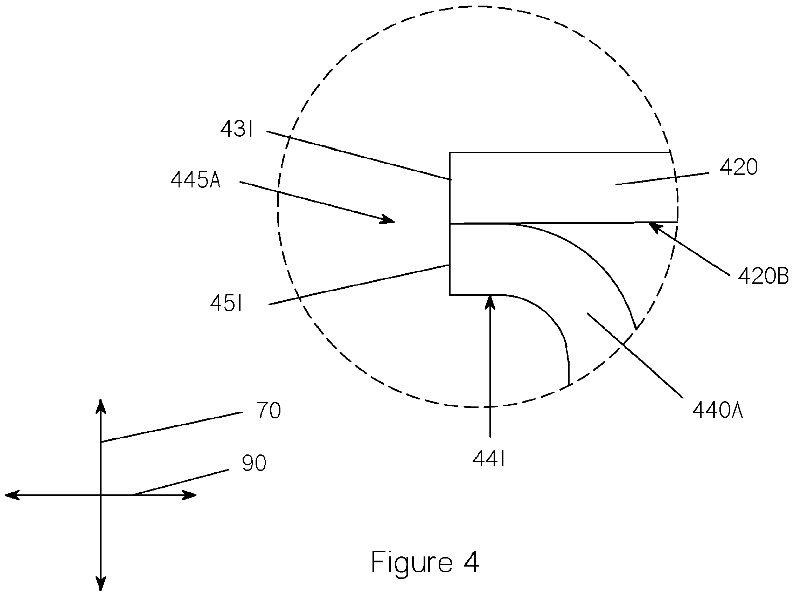

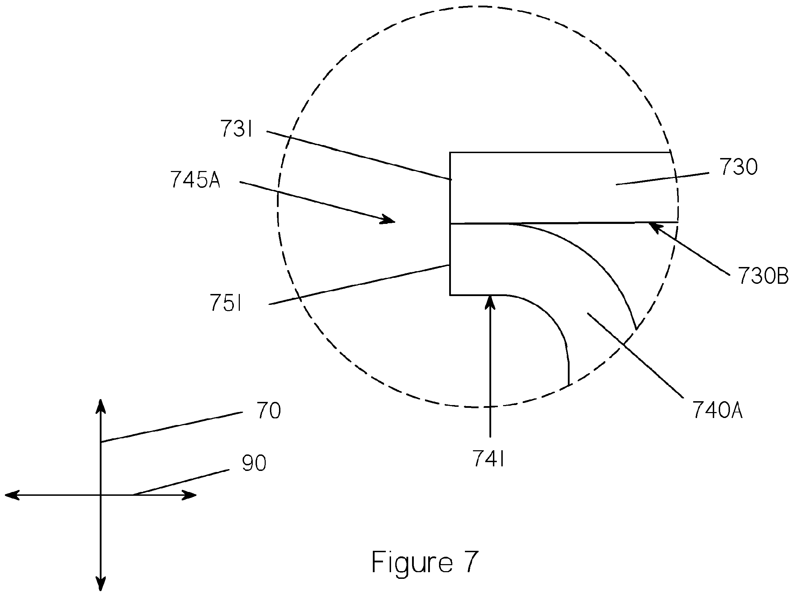

The absorbent articles of the present invention comprise a chassis having a topsheet, a backsheet, and an absorbent core disposed between the topsheet and the backsheet. The absorbent articles further comprise a pair of side flaps attached to the chassis. In some embodiments as discussed with regard to Figures 1-3, side flaps may be attached to an outer-facing surface of the chassis. In some embodiments, as discussed with regard to Figures 4-6, side flaps may be attached to an outer-facing surface of the chassis wherein, an outer-facing surface of the topsheet forms a portion of the outer-facing surface of the chassis. Still in other embodiments, as discussed with regard to Figures 7-9, side flaps may be attached to an outer-facing surface of the chassis wherein, an outer-facing surface of the backsheet forms a portion of the outer-facing surface of the chassis. Similarly, in some embodiments, a body-facing surface of the topsheet may form a portion of a body-facing surface of the chassis.

The absorbent articles of the present invention can facilitate the application of the absorbent article to an undergarment of the wearer as described herein. For example, the attachment of the side flaps to the chassis can create a fold axis which can more closely follow the contour of the user's undergarment than side flaps on commercially available absorbent articles. This can improve the comfort and fit that the user experiences during use. Additionally, the attachment of the side flaps to the chassis of the absorbent article can provide guides to the consumers with regard to centering the absorbent article on the undergarment. Last, the orientation of the absorbent article either in its package or once removed from its package, can visually signal to the consumer the benefit of enhanced protection. Flange seams, described hereafter, can communicate a benefit of additional protection to the consumer.

For ease of reference, as shown in Figure 1, for absorbent articles constructed in accordance with the present invention, a longitudinal direction 80 shall refer to a direction extending generally parallel to a maximum length of an absorbent article and a lateral direction 90 shall refer to a direction extending generally perpendicular to the longitudinal direction 80 and in the same plane or a plane parallel to a plane in which the absorbent article is disposed in a flat, unfolded state. The lateral direction 90 is generally parallel to a width of the absorbent article as measured between opposite longitudinal edges of the absorbent article. A vertical direction 70 is shall refer to a direction extending generally perpendicular to the lateral direction 90 and the longitudinal direction 80 and generally parallel to the thickness of the absorbent article.

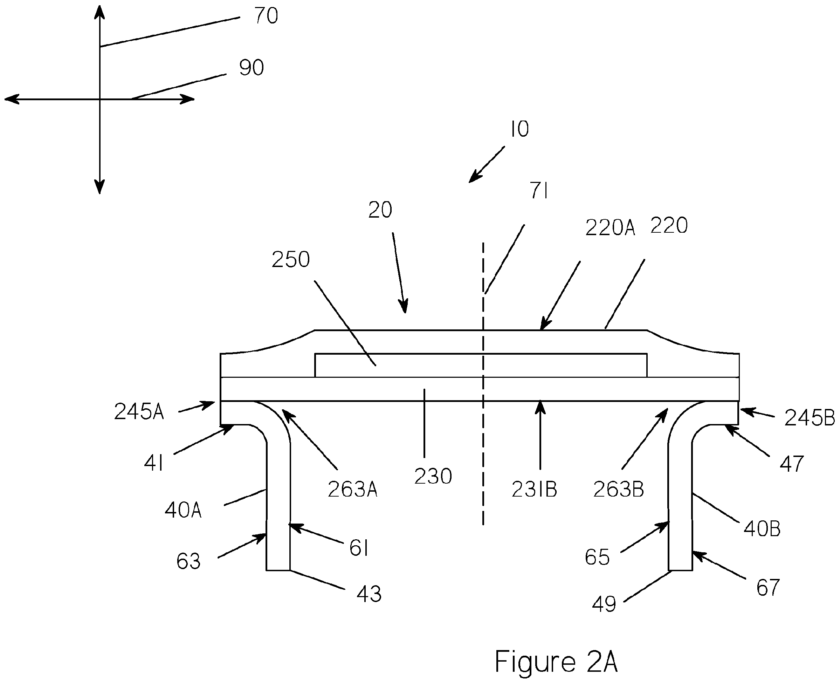

Referring to Figures 1 and 2A, an absorbent article 10 is depicted with a longitudinal axis 81, a lateral axis 91, and a vertical axis 71. The longitudinal axis 81 is generally parallel to the longitudinal direction 80, the lateral axis 91 is generally parallel to the lateral direction 90, and the vertical axis 71 is generally parallel to the vertical direction 70. The longitudinal axis 81 bisects a width of the absorbent article 10 as measured from a first longitudinal edge 30A of a periphery 30 to a second longitudinal edge 30B of the periphery 30, and the lateral axis 91 bisects a maximum length of the absorbent article 10. The vertical axis 71 bisects the width of the absorbent article 10 in cross section.

The absorbent article 10, constructed in accordance with the present invention, is shown in an unfolded state. The absorbent article 10 comprises a chassis 20 which further comprises a topsheet 220, a backsheet 230, and an absorbent core 250 between the topsheet 220 and the backsheet 230. The chassis 20 defines the periphery 30 comprising the first longitudinal edge 30A and the second longitudinal edge 30B. As shown, in some embodiments, each of the first and second longitudinal edges 30A and 30B may include curvature to provide a comfortable wearing experience for the user.

As shown in Figure 1, the absorbent article 10 further comprises a pair of side flaps, 40A and 40B, which are attached to the chassis 20. As shown, each of the side flaps 40A and 40B extend generally laterally outward from the periphery 30 of the chassis 20.

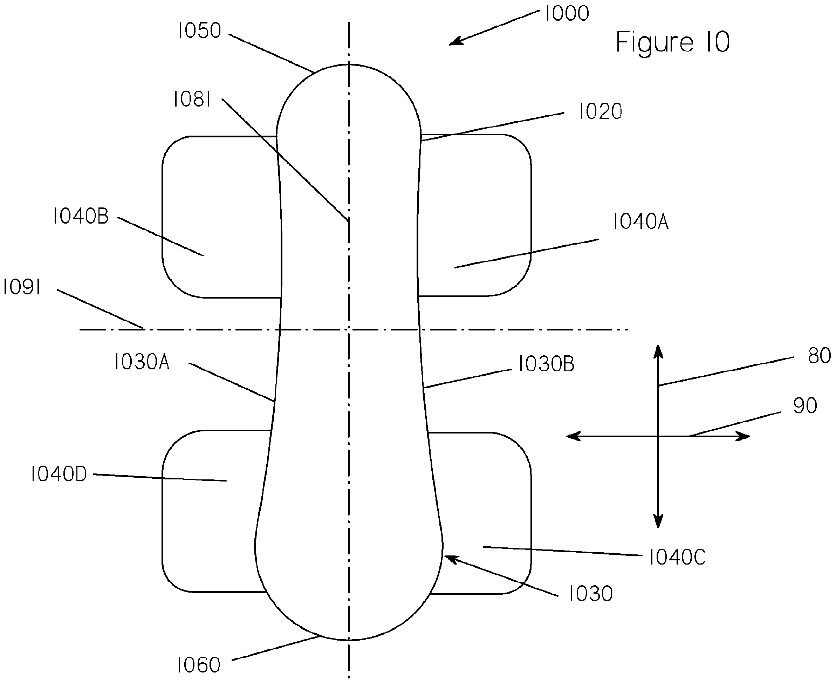

The side flaps 40A and 40B may be positioned along the periphery 30 of the chassis 20 in any suitable location. In some embodiments, the side flaps 40A and 40B may be positioned near a first end 50 of the chassis 20. In some embodiments, the side flaps 40A and 40B may be positioned near a second end 60 of the chassis 20. Still in other embodiments, the side flaps 40A and 40B may be positioned near the lateral axis 91. Still in other embodiments, the side flaps 40A and 40B may be positioned on the lateral axis 91. Still in other embodiments, more than one pair of side flaps may be utilized as discussed hereafter with regard to Figure 10.

Referring to Figure 2A, the side flaps 40A and 40B are shown hanging downward. In one embodiment, a first surface 61 of side flap 40A and a first surface 65 of side flap 40B may each be joined to an outer-facing surface 231B of the backsheet 230 forming flange seams 245A and 245B, respectively. The flange seams 245A and 245B may form guides 263A and 263B, respectively, which can help a user center the absorbent article 10 on their undergarment when affixing the absorbent article 10 thereto.

The side flaps 40A and 40B comprise an attached area 41 and 47, respectively, and free ends 43 and 49, respectively. The attached areas 41 and 47 are joined to the chassis 20 and form a portion of the flange seams 245A and 245B, respectively. The free ends 43 and 49 are typically positioned subjacent to the undergarment of the user during use. For example, the side flap 40A comprises the first surface 61 and side flap 40B comprises the first surface 65 and each side flap 40A and 40B comprises a second surface 63 and 67, respectively. The first surface 61 of the side flap 40A may comprise an adhesive for adherence to the undergarment of the user. Additionally, the first surface 65 of the side flap 40B may also comprise adhesive for adherence to the undergarment of the user.

During the application of the absorbent article 10, the side flaps 40A and 40B can fold about an axis which is immediately adjacent to their respective attached areas 41 and 47. The fold axis is created via the flange seams 245A and 245B and/or the attached areas 41 and 47. The fold axis can further facilitate the application of the side flaps 40A and 40B to the undergarment of the wearer. Unlike the side flaps on conventional absorbent articles which have no predefined fold axis, the user is often left guessing or forming, on an ad hoc basis, a fold axis which can introduce stresses into the side flap. For the embodiments of the present invention, the fold axis is created via the flange seams 245A and 245B and attached areas 41 and 47. And, in some embodiments, the attached areas 41 and 47 and can be configured to closely follow the contour of the undergarment of the wearer as discussed hereafter.

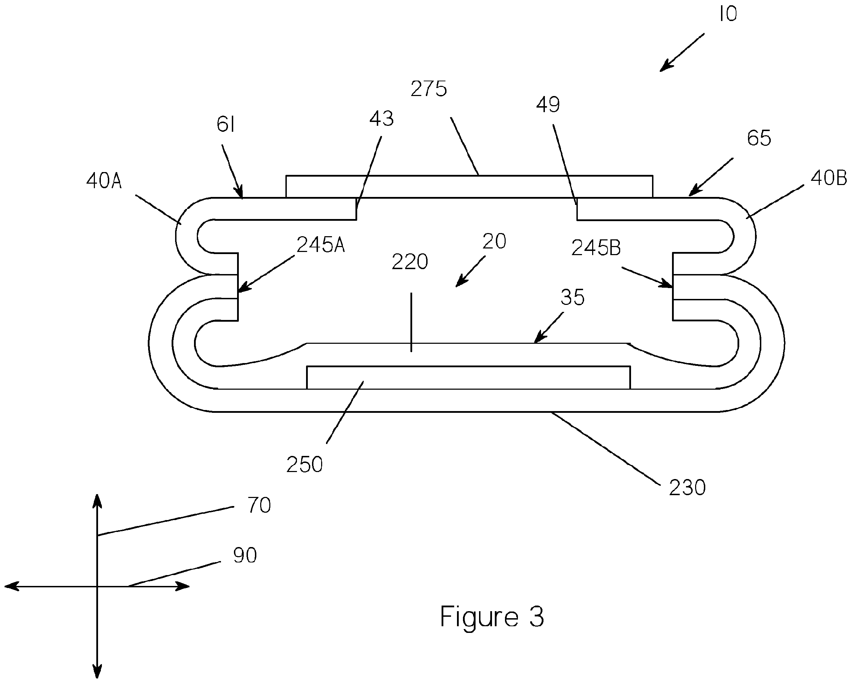

When initially removed from its outer packaging, the absorbent article 10 may be folded in half, in thirds or more. Additionally, a first release layer may be provided covering adhesive on the backsheet 230, and a second release layer may be provided covering adhesive on the side flaps 40A and 40B. For ease of viewing, neither the first release layer nor the second release layer is shown. However, the release layers will be discussed hereafter with regard to Figure 3 and further herein.

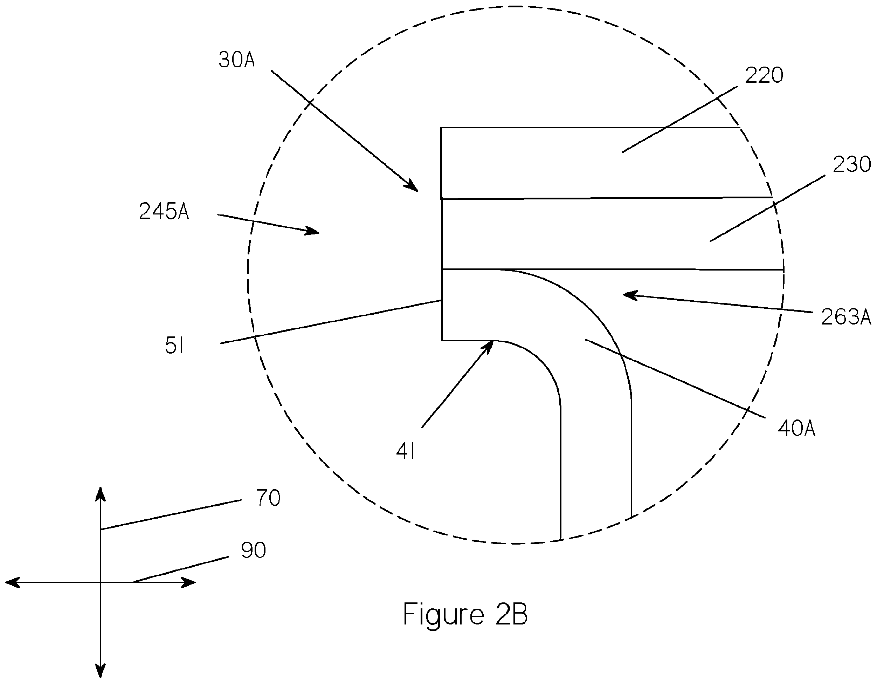

With regard to Figures 2A and 2B, the chassis 20 comprises the first longitudinal edge 30A which as shown may be coterminous with a side flap end 51. As shown, the first longitudinal edge 30A may comprise an end portion of the topsheet 220 and an end portion of the backsheet 230 which may be coterminous in some embodiments. In some embodiments, the topsheet 220 may extend laterally and/or longitudinally outboard more than the backsheet 230 in at least a portion of the periphery 30 (shown in Figure 1) of the absorbent article 10. In some embodiments, the backsheet 230 may extend laterally and/or longitudinally outboard more than the topsheet 220 in at least a portion of the periphery 30 of the absorbent article 10. Although not shown, the second longitudinal edge 30B (shown in Figure 1) may be similarly configured.

As shown, the flange seam 245A may comprise a portion of the first longitudinal edge 30A and the attached area 41 of side flap 40A. Similarly, flange seam 245B may comprise a portion of the second longitudinal edge 30B (shown in Figure 1) and the attached area 47 of side flap 40B.

In some embodiments, the attached area 41 of side flap 40A may extend from the side flap end 51 laterally inboard by about 5 mm to about 10 mm. In some embodiments, the attached area maybe less than 5 mm. However, in some embodiments, the attached area 41 may begin laterally inboard from the side flap end 51 by about 2 mm to about 5 mm. Similarly, the attached area 47 of side flap 40B may be similarly configured as the attached area 41.

During processing, after creation of the chassis 20, the chassis 20 may be cut to provide the curved first and second longitudinal edges 30A and 30B (shown in Figure 1). The side flaps 40A and 40B comprise discrete elements which are attached to the chassis 20 of the absorbent article 10. For the sake of clarification, in such embodiments, the side flaps 40A and 40B are not integrally formed from the topsheet 220, the backsheet 230, and/or combinations thereof. In such embodiments, the material of each the side flaps 40A and 40B may be attached to the chassis 20 prior to the cutting process of the chassis 20. So, during the cutting process as mentioned above, the attached areas 41 and 47 and/or their respective side flap ends, e.g. 51, may be subjected to the cutting process as well. This can result in the attached areas 41 and 47 of their respective side flaps 40A and 40B and/or the side flap ends, e.g. 51, following the curved periphery of the chassis 20. This can provide the user with additional comfort. Additionally, in such embodiments, because the attached areas 41 and 47 and/or their respective side flap ends, e.g. 51, follow the curvature of the periphery 30, many of the stresses placed on conventional side flaps by a user's undergarment are alleviated if not eliminated without additional processing, e.g. stretching, activation, or differential extensibility of nonwoven material for side flaps. Stresses on conventional side flaps are discussed in detail in U.S. Patent No. 5,354,400, issued to Lavash et al.

Additional embodiments are contemplated where each of the side flaps 40A and 40B is attached to the chassis 20 such that the side flaps 40A and 40B extend laterally outboard of the first and second longitudinal edges 30A and 30B (shown in Figure 1) of the absorbent article 10. In such embodiments, each of the side flaps 40A and 40B may be joined to the backsheet 230 and topsheet 220 such that each of the side flaps 40A and 40B is attached to both the outer-facing surface 231B of the backsheet 230 and a body-facing surface 220A of the topsheet 220.

The side flaps 40A and 40B may further comprise adhesive which is disposed on the first surface 61 of the side flap 40A and the first surface 65 of the side flap 40B. The adhesive can help adhere the side flaps 40A and 40B to the undergarment of the wearer during use. Suitable adhesives are discussed hereafter.

Referring to Figure 3, the absorbent article 10 is shown in a flat state. When applying an absorbent article to their undergarment, a user may remove a release liner (not shown) which covers a pressure sensitive adhesive disposed on the backsheet 230 of the absorbent article 10. After removing this release liner, the user may depress the absorbent article 10 in place on their undergarment thereby adhering the absorbent article 10 to the undergarment via the adhesive on the backsheet 230.

In order to prevent premature attachment of the side flaps 40A and 40B to the skin of the wearer or some unintended surface, a release liner 275 may be provided which covers the adhesive on the first surface 61 of side flap 40A and adhesive on the first surface 65 of side flap 40B until the user is ready to adhere the side flaps 40A and 40B to their undergarment. The release liner 275 can facilitate the application of the absorbent article 10 to the undergarment of the wearer by allowing the user to first adhere the absorbent article 10 to their undergarment via the adhesive on the backsheet 230 while the side flaps 40A and 40B are conveniently held out of the way via the release liner 275. As shown, the free ends 43 and 49 are positioned vertically superjacent a body-facing surface 35 of the chassis 20 prior to the removal of the release liner 275 and/or prior to use. Once the absorbent article 10 is adhered to the undergarment, the user can then remove the release liner 275 and adhere the side flaps 40A and 40B to their undergarment.

Additionally, prior to use as shown, the flange seams 245A and 245B or a portion thereof may be positioned vertically superjacent to the body-facing surface 35 of the chassis 20. In such embodiments, although the guides 263A and 263B (shown in Figure 2A) may not be available at the time of the initial application of the absorbent article 10 to the undergarment of the wearer, such arrangements of the side flaps 40A and 40B still assist the user with application of the absorbent article 10. For example, as stated previously, the fold axis created via the flange seams 245A and 245B and the attached areas 41 and 47 (shown in Figure 2A) can facilitate the application of the side flaps 40A and 40B to the undergarment of the wearer as discussed previously.

Additional embodiments are contemplated where the application of the absorbent article 10 to the undergarment of the user via the guides 263A and 263B (each shown in Figure 2) is available. In such embodiments, the flange seams 245A and 245B may be positioned laterally outboard of the absorbent core 250 prior to use with a release liner holding side flaps 40A and 40B in the previously described position. Such arrangements can ensure that the guides 263A and 263B are present during the application of the absorbent article 10 to the undergarment of the user via the adhesive disposed on the backsheet 230.

Side flaps of the present invention may be attached to the chassis in a number of different configurations - some examples of which are discussed hereafter. Each of the embodiments described can facilitate the application of an absorbent article to a user's undergarments via guides formed by flange seams created by the attachment between side flaps and the chassis and/or via the fold axis created by the flange seams and attached areas. Additionally, each of the embodiments described below may utilize side flaps formed from discrete elements and be attached to a chassis as heretofore described regarding side flaps comprising discrete elements. Embodiments are contemplated where the side flaps may comprise an integral construction, e.g. formed from a portion of the topsheet, backsheet, or laminates / combinations thereof.

As shown in Figure 4, in some embodiments, a side flap 440A may be attached to a topsheet 420 on an outer-facing surface 420B of the topsheet 420. The attachment of the topsheet 420 and the side flap 440A may form a flange seam 445A which comprises an attached area 441 of the side flap 440A. As shown, a topsheet end 431 may be coterminous with a side flap end 451. In some embodiments, the attached area 441 of side flap 440A may extend from the side flap end 451 laterally inboard by about 5 mm to about 10 mm. In some embodiments, the attached area 441 may be less than about 5 mm. However, in some embodiments, the attached area 441 may begin laterally inboard from the side flap end 451 by about 2 mm to about 5 mm.

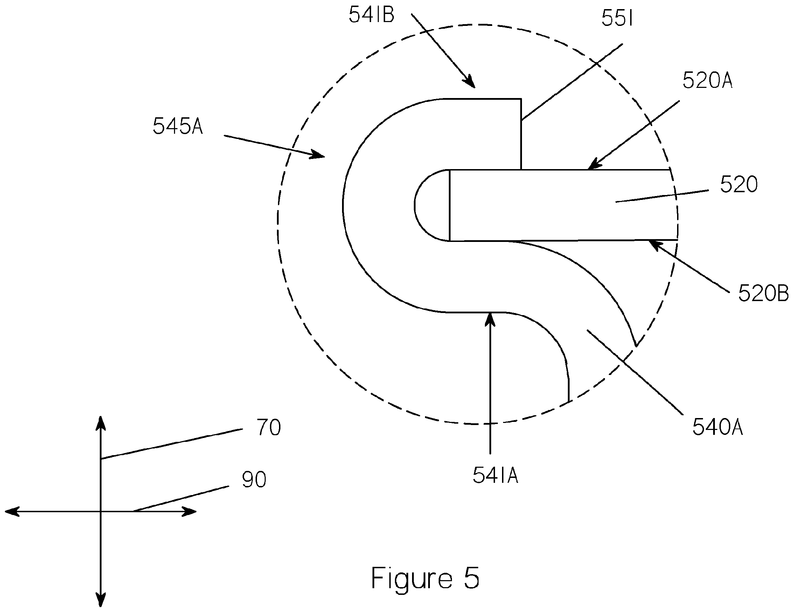

Alternatively, in some embodiments, as shown in Figure 5, a side flap 540A may be attached to a topsheet 520 in a plurality of locations to form a flange seam 545A. For example, the side flap 540A may be attached to a body-facing surface 520A and an outer-facing surface 520B of the topsheet 520. In such embodiments, during use, a side flap end 551 or a portion the side flap 540A may extend in a direction generally parallel to the vertical direction 70. For such embodiments, it is contemplated that the side flap end 551 or portion of the side flap 540A may form a cuff / barrier like structure which could assist in precluding bodily exudates from soiling the garment of the wearer. The flange seam 545A may comprise attached areas 541A attached to the outer-facing surface 520B of the topsheet 520 and 541B where the side flap 540A is attached to the body-facing surface 520A of the topsheet 520.

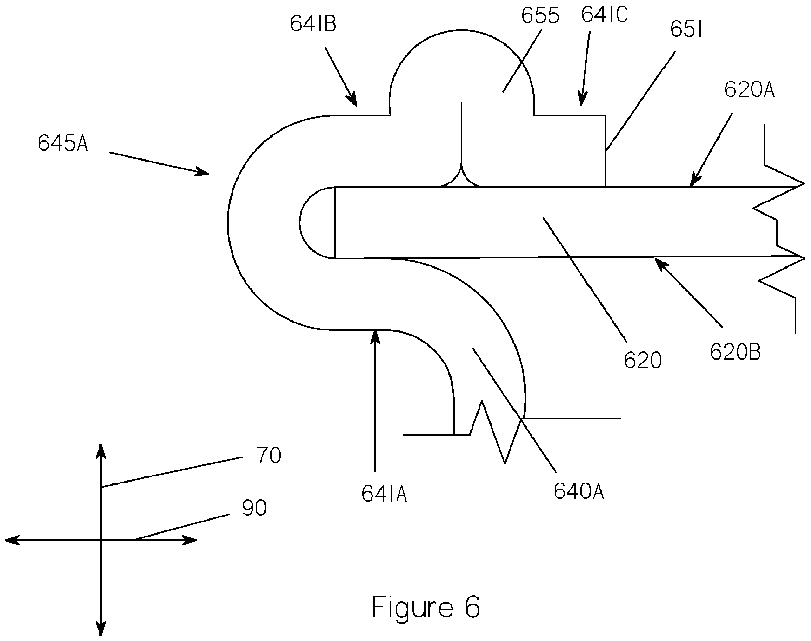

In another embodiment, as shown in Figure 6, a side flap 640A may be attached to an outer-facing surface 620B and a body-facing surface 620A of a topsheet 620 forming a flange seam 645A. Flange seam 645A may comprise attached areas 641A, 641B, and 641C. Additionally, as shown, the side flap 640A may comprise a cuff 655. The side flap 640A may be joined to itself to form the cuff 655 and/or the cuff 655 may be formed via the spacing of the attached areas 641B and 641C and an excess side flap material therebetween. Additional side flaps may be constructed similarly.

The cuff 655 may be disposed on the body-facing surface 620A of the topsheet 620. Similarly, a side flap end 651 may form a cuff / barrier as well. Depending on the material utilized for the side flap 640A, the side flap end 651 and the cuff 655 can be made to be substantially hydrophobic thereby making the cuff 655 and the side flap end 651 substantially liquid impermeable.

Regarding Figure 7, in some embodiments, a side flap 740A may be attached to an outer-facing surface 730B of a backsheet 730. The attachment of the backsheet 730 and the side flap 740A may form a flange seam 745A which comprises an attached area 741 of the side flap 740A. As shown, a backsheet end 731 may be coterminous with a side flap end 751. In some embodiments, the attached area 741 of side flap 740A may extend from the side flap end 751 laterally inboard by about 5 mm to about 10 mm. In some embodiments, the attached area 741 may extend less than about 5mm. However, in some embodiments, the attached area 441 may begin laterally inboard from the side flap end 751 by about 2 mm to about 5 mm.

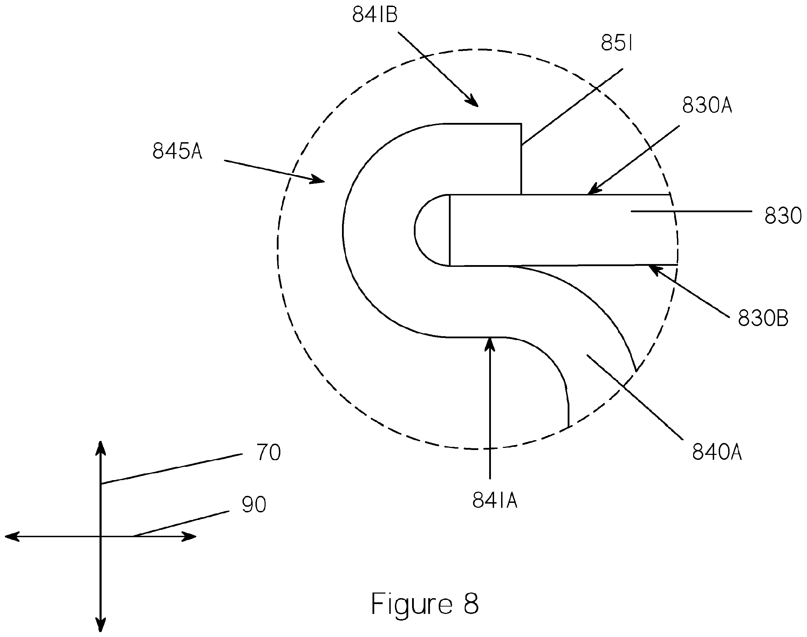

In some embodiments, as shown in Figure 8, a side flap 840A may be attached to a backsheet 830 in a plurality of locations to form a flange seam 845A. For example, the side flap 840A may be attached to a body-facing surface 830A and an outer-facing surface 830B of the backsheet 830. In such embodiments, during use, a side flap end 851 or a portion of the material of the side flap 840A may extend in a direction generally parallel to the vertical direction 70. For such embodiments, it is contemplated that the side flap end 851 or portion of the material of the side flap 840A may form a cuff / barrier like structure which could assist in precluding bodily exudates from soiling the garment of the wearer. The flange seam 845A may comprise attached areas 841A and 841B where the side flap 840A is attached to the outer-facing surface 830B and the body-facing surface 830A, respectively, of the backsheet 830.

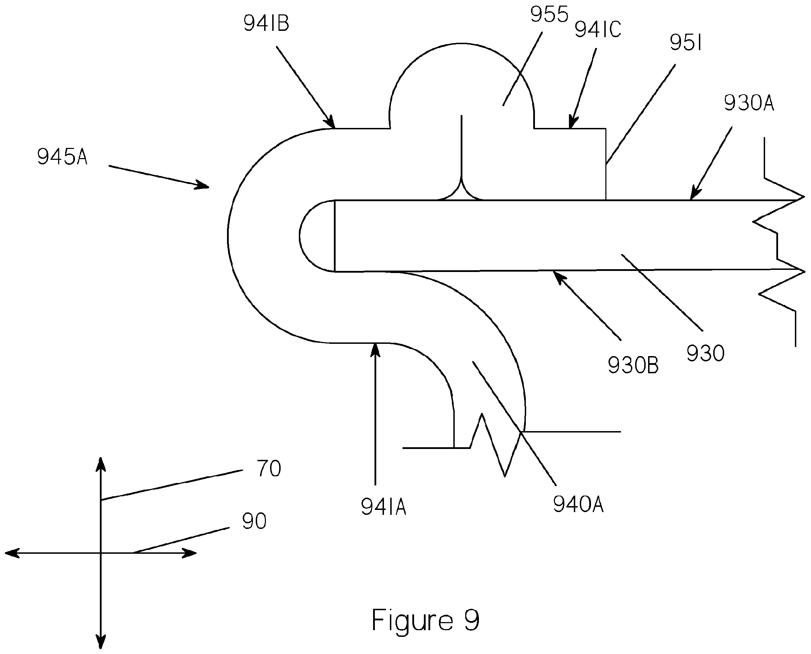

In another embodiment, as shown in Figure 9, a side flap 940A may be attached to an outer-facing surface 930B and a body-facing surface 930A of a backsheet 930 forming a flange seam 945A. Flange seam 945A may comprise attached areas 941A, 941B, and 941C. Additionally, as shown, the side flap 940A may comprise a cuff 955 which comprises the side flap 940A. The side flap 940A may be joined to itself to form the cuff 955. In some embodiments, attached areas 941B and 941C may be spaced such that material for the side flap 940A forms the cuff. In such embodiments, the material for the side flap may or may not be bonded to itself to form the cuff 955.

The cuff 955 may be disposed on the body-facing surface 920A of the backsheet 930. Similarly, a side flap end 951 may form a cuff / barrier as well. Depending on the material utilized for the side flap 940A, the side flap end 951 and the cuff 955 can be made to be substantially hydrophobic thereby making the cuff 955 and the side flap end 951 substantially liquid impermeable.

The configuration provided for side flaps 440A, 540A, 640A, 740A, 840A, and 940A may be applied to additional side flaps of an absorbent article. Similarly, the configurations described with regard to side flaps 440A, 540A, 640A, 740A, 840A, and 940A may be applied to the side flaps 40A and 40B discussed previously with regard to Figures 1-3. For example, side flaps 40A and 40B may be configured such that each is attached to the chassis on the outer-facing surface and the body-facing surface of the chassis. Similarly, side flaps 40A and/or 40B can be configured to provide cuffs on the body-facing surface of the chassis.

Referring to Figure 10, additional embodiments are contemplated where an absorbent article 1000 comprises a plurality of pairs of side flaps 1040A, 1040B, 1040C and 1040D is shown in an unfolded state. As shown, the absorbent article 1000 comprises a longitudinal axis 1081 that is generally parallel with the longitudinal direction 80 and a lateral axis 1091 generally parallel with the lateral direction 90. Similarly to the absorbent article 10 (shown in Figure 1), when initially removed from its package, the absorbent article 1000 may be folded in half, in thirds or more.

The absorbent article 1000 may comprise a chassis 1020 defining a periphery 1030 having a first longitudinal edge 1030A and a second longitudinal edge 1030B. As shown, each of the side flaps 1040A, 1040B, 1040C, and 1040D extend generally laterally outward from the periphery 1030 of the chassis 1020. Additionally, side flaps 1040A, 1040B, 1040C, and 1040D may be positioned any suitable location along the curved periphery 1030 of the absorbent article 1000 and held in place by a first release layer (not shown).

The side flaps 1040A, 1040B, 1040C, and 1040D may be positioned along the periphery 1030 of the chassis 1020 in any suitable location. In some embodiments, at least one pair of the side flaps 1040A, 1040B, 1040C, and 1040D may be positioned near a first end 1050 of the chassis 1020. In some embodiments, at least one pair of the side flaps 1040A, 1040B, 1040C, and 1040D may be positioned near a second end 1060 of the chassis 1020. Still in other embodiments, at least one pair of the side flaps 1040A, 1040B, 1040C, and 1040D may be positioned near the lateral axis 1091. Still in other embodiments, at least one pair of the side flaps 1040A, 1040B, 1040C, and 1040D may be positioned on the lateral axis 1091. Each of the side flaps 1040A, 1040B, 1040C and 1040D may be configured similarly to the side flaps described herein.

As stated previously, absorbent articles in accordance with the present invention can provide at least one of many benefits to consumers. However, processing of absorbent articles in accordance with the present invention can present some unique challenges. Side flaps on conventional sanitary napkins are unbiased. As such, processes for folding the side flaps above their respective topsheets are relatively established. In contrast, the wings of the absorbent articles of the present invention are biased against being held superjacent to their respective topsheets. As such, conventional processing may not accomplish the desired absorbent article for packaging.

Any suitable material may be utilized for the side flaps disclosed herein. Some examples include but are not limited to, nonwovens, nonwoven laminates, film-nonwoven laminates, elastomeric films laminate, elastomeric nonwovens laminate, elastomeric film-nonwoven laminates, differentially extensible substrates or laminates thereof, textile or woven materials such as cotton fabrics, elastic materials, stretchable materials, and/or combinations thereof. Embodiments are contemplated where at least one of the side flaps comprises an antibacterial and/or antimicrobial additive / composition or bacterial / microbial growth inhibitors. An example of such a composition is described in U.S. Patent No. 7,790,947. For example, silver ions may be added to the material of at least one of the side flaps. In some embodiments, at least one of the side flaps may be breathable allowing air / vapor to permeate therethrough but not allowing liquid to pass therethrough. In some embodiments, at least one of the side flaps may be transparent or translucent or may have portions which are transparent and portions which are translucent. In some embodiments, at least one of the side flaps may comprise a friction reducing composition and / or a lotion and/or a sensate. In some embodiments, the lotion may provide the function of the friction reducing composition in addition to other benefits. Suitable lotions and sensates are disclosed in U.S. Patent Application Publication No. 2012/089110; U.S. Patent Application Publication No. 2011/070277; and U.S. Patent No. 8,357,445. Examples of additional lotions and/or sensates are described in U.S. Pat. No, 6,570,054 and U.S. patent application Ser. No. 12/974,674. In some embodiments, the side flaps may comprise materials which comprise compositions which enhance its hydrophobicity. Some examples include hydrophobic additive formulations. Hydrophobic additive formulations and methods for incorporating them in nonwoven webs are described by Catalan in US applications publication Nos. 2006/0189956 filed on Feb. 18, 2005, and 2005/0177123 filed on Feb. 10 2005, and in U.S. application Ser. No. 12/691,929 filed on Jan. 22, 2010, and U.S. application Ser. No. 12/691,934 filed on Jan. 22, 2010 both to J J Tee et al. that are all assigned to The Procter and Gamble Company. Some suitable, but not limiting, hydrophobic materials used as hydrophobic surface coatings and/or hydrophobic melt additives may comprise one or more silicone polymers that are also substantially free of aminosilicones, Suitable silicone polymers are selected from the group of silicone MQ resins, polydimethysiloxanes, crosslinked silicones, silicone liquid elastomers, and combinations thereof. Typically, the molecular weight of such silicone polymers should be at least 4000 MW. However, the molecular weight of such silicone polymers may be at least 10,000 MW, at least 15,000 MW, at least 20,000 MW, or at least 25,000 MW. Suitable polydimethylsilosxanes are selected from the group consisting of vinyl-terminated polydimethsiloxanes, methyl hydrogen dimethylsiloxanes, hydroxyl-terminated polydimethysiloxanes, organo-modified polydimethylsiloxanes, and combinations thereof. Alternatively, fluorinated polymers may also be used as the hydrophobic surface coatings and/or the hydrophobic melt additives. Suitable fluorinated polymers are selected from the group of telomers and polymers containing tetrafluoroethylene and/or perfluorinated alkyl chains. For instance, fluorinated surfactants, which are commercially available from Dupont under the tradename Zonyl ®, are suitable for use herein.

In some embodiments, the side flaps may comprise "capillary channel fibers." Such fibers can be solid or hollow, and they can be tri-lobal, delta-shaped, and are preferably fibers having capillary channels on their outer surfaces. The capillary channels can be of various cross-sectional shapes such as "U-shaped", "H-shaped", "C-shaped" and "V-shaped". One suitable example of a capillary channel fiber is T-401, designated as 4DG fiber available from Fiber Innovation Technologies, Johnson City, Tenn. T-401 fiber is a polyethylene terephthalate (PET polyester).

In some embodiments, the side flaps may comprise microfibers. Meltblown fibers are microfibers which may be continuous or discontinuous and are generally smaller than 10 microns in average diameter. In some embodiments, the side flaps may comprise Coolmax ® fibers.

Any suitable adhesive may be utilized on backsheets and/or side flaps disclosed herein. The adhesive can be hot-melt adhesive material capable of establishing a temporary bond with the undergarment material. A suitable adhesive is the composition designated HL-1491 XZP commercially available from H. B. Fuller, Toronto, Ontario, Canada, a composition designated as H2031 available from Bostik, a composition designated as NS34-2823 as manufactured by National Starch and Chemical of Bridgewater, N.J.

The adhesive can be applied to the backsheet at levels between about 9 gsm to about 20 gsm, in some embodiments. The adhesive applied to the backsheet may be applied in discrete strips or may cover a substantial portion of the backsheet. Regarding the adhesive for the side flaps, the adhesive above may be suitable and an additional adhesive which is suitable is designated as LA203 available from Savare Specialty Adhesives or the composition designated as 1461 available from H.B. Fuller. The adhesive for the side flaps may be applied at levels of between about 13 gsm to about 38 gsm.

Any suitable release liners can be used in conjunction with the present invention. Some examples of suitable release liners include those disclosed in U.S. Patent No. 4,556,146, and U.S. Patent Application Publication No. 2011/0202029. The releasable cover may be a silicone coated release liner, a plastic film or any other easily removable cover. The releasable cover may be in a single piece or in a multitude of pieces, e.g. to cover the individual adhesive areas. It also can perform other functions such as providing individualized packaging for the article or provide a disposal function. Any commercially available release liner or film may be used. Some suitable examples include BL 30 MG-A SILOX EI/O, BL 30 MG-A SILOX 4 P/O available from Akrosil Corporation, and M&W films available from Gronau in Germany, under the code X-5432.

The methods of the invention can be used with any suitable feminine hygiene article. Suitable absorbent articles include any type of structures, from a single absorbent layer to more complex multi layer structures. Certain absorbent articles typically include a fluid pervious topsheet, a backsheet, which may be fluid impervious and/or may be water vapour and/or gas pervious, and an absorbent element often called "core" comprised there between.

In general the topsheet is operatively permeable to the liquids that are intended to be held or stored by the absorbent article, and the backsheet may be substantially impermeable or otherwise operatively impermeable to the intended liquids. The absorbent article may also include other components, such as a secondary topsheet, liquid wicking layers, liquid distribution layers, barrier layers, and the like, as well as combinations thereof. Suitable topsheets, backsheets, side flaps and absorbent core materials for use in conjunction with the present invention are discussed hereafter. Additionally, suitable joining methods for topsheets, backsheets, side flaps, and optionally the absorbent core are also discussed hereafter.

The topsheet may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be included of natural fibers (e.g., wood or cotton fibers), synthetic fibers (e.g., polymeric fibers such as polyester, polypropylene, or polyethylene fibers) or from a combination of natural and synthetic fibers. In one embodiment, the topsheet may be made of a hydrophobic material to isolate the wearer's skin from liquids which have passed through the topsheet. If the topsheet is made of a hydrophobic material, at least the upper surface of the topsheet is treated to be hydrophilic so that liquids will transfer through the topsheet more rapidly. This diminishes the likelihood that body exudates will flow off the topsheet rather than being drawn through the topsheet and being absorbed by the absorbent core. In one embodiment, the topsheet can be rendered hydrophilic by treating it with a surfactant. Suitable methods for treating the topsheet with a surfactant include spraying the topsheet material with the surfactant and immersing the material into the surfactant.

The topsheet can include an apertured formed film. Apertured formed films can be used for the topsheet because they are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Pat. No. 3,929,135, entitled "Absorptive Structures Having Tapered Capillaries", issued to Thompson on Dec. 30, 1975; U.S. Pat. No. 4,324,246 entitled "Disposable Absorbent Article Having A Stain Resistant Topsheet", issued to Mullane, et al. on Apr. 13, 1982; U.S. Pat. No. 4,342,314 entitled "Resilient Plastic Web Exhibiting Fiber-Like Properties", issued to Radel, et al. on Aug. 3, 1982; U.S. Pat. No. 4,463,045 entitled "Macroscopically Expanded Three-Dimensional Plastic Web Exhibiting Non-Glossy Visible Surface and Cloth-Like Tactile Impression", issued to Ahr, et al. on Jul. 31, 1984; and U.S. Pat. No. 5,006,394 "Multilayer Polymeric Film" issued to Baird on Apr. 9, 1991.

The absorbent core can be any absorbent member which is generally compressible, conformable, non-irritating to the wearer's skin, and capable of absorbing and retaining body fluids. The absorbent core may be manufactured in a wide variety of sizes and shapes (e.g., rectangular, hourglass, "T" -shaped, asymmetric, etc.) and from a wide variety of liquid-absorbent materials commonly used in disposable pull-on garments and other absorbent articles such as commuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

The configuration and construction of the absorbent core may vary (e.g., the absorbent core may have varying caliper zones, a hydrophilic gradient, a superabsorbent gradient, or lower average density and lower average basis weight acquisition zones; or may include one or more layers or structures). Further, the size and absorbent capacity of the absorbent core may also be varied to accommodate wearers ranging from infants through adults. However, the total absorbent capacity of the absorbent core should be compatible with the design loading and the intended use of the absorbent article.

The absorbent core may include other optional components. One such optional component is the core wrap, i.e., a material, typically but not always a nonwoven material, which either partially or totally surrounds the core. Suitable core wrap materials include, but are not limited to, cellulose, hydrophilically modified nonwoven materials, perforated films and combinations thereof.

The backsheet can comprise a liquid impervious film. The backsheet can be impervious to liquids (e.g., body fluids) and can be typically manufactured from a thin plastic film. However, typically the backsheet can permit vapours to escape from the disposable article. In an embodiment, a microporous polyethylene film can be used for the backsheet. A suitable microporous polyethylene film is manufactured by Mitsui Toatsu Chemicals, Inc., Nagoya, Japan and marketed in the trade as PG-P.

One suitable material for the backsheet can be a liquid impervious thermoplastic film having a thickness of from about 0.012 mm (0.50 mil) to about 0.051 mm (2.0 mils), for example including polyethylene or polypropylene. Typically, the backsheet can have a basis weight of from about 5 g/m² to about 35 g/m². However, it should be noted that other flexible liquid impervious materials may be used as the backsheet. Herein, "flexible" refers to materials which are compliant and which will readily conform to the general shape and contours of the wearer's body.

The backsheet can be typically positioned adjacent a outer-facing surface of the absorbent core and can be joined thereto by any suitable attachment device known in the art. For example, the backsheet may be secured to the absorbent core by a uniform continuous layer of adhesive, a patterned layer of adhesive, or an array of separate lines, spirals, or spots of adhesive. Illustrative, but non-limiting adhesives, include adhesives manufactured by H. B. Fuller Company of St. Paul, Minn., U.S.A., and marketed as HL-1358J. An example of a suitable attachment device including an open pattern network of filaments of adhesive is disclosed in U.S. Pat. No. 4,573,986 entitled "Disposable Waste-Containment Garment", which issued to Minetola et al. on Mar. 4, 1986. Another suitable attachment device including several lines of adhesive filaments swirled into a spiral pattern is illustrated by the apparatus and methods shown in U.S. Pat. No. 3,911,173 issued to Sprague, Jr. on Oct. 7, 1975; U.S. Pat. No. 4,785,996 issued to Ziecker, et al. on Nov. 22, 1978; and U.S. Pat. No. 4,842,666 issued to Werenicz on Jun. 27, 1989. Alternatively, the attachment device may include heat bonds, thermal fusion bonds, pressure bonds, ultrasonic bonds, dynamic mechanical bonds, or any other suitable attachment device or combinations of these attachment devices. The backsheet may be additionally secured to the topsheet by any of the above-cited attachment devices / methods.

The absorbent article may also include such other suitable features as are known in the art including, but not limited to, re-closable fastening system, lotion, acquisition layers, distribution layers, wetness indicators, sensors, elasticized waist bands and other similar additional elastic elements and the like, belts and the like, waist cap features, containment and aesthetic characteristics and combinations thereof.

## Claims

1. An absorbent article (10, 1000) having a longitudinal axis (81, 1081), a lateral axis (91, 1091) extending generally perpendicular to the longitudinal axis, and a vertical axis (71) extending generally perpendicular to the longitudinal axis and the lateral axis, the absorbent article further comprising:
a chassis (20, 1020) comprising a topsheet (220, 420, 520, 620), a backsheet (230, 730, 830, 930), and an absorbent core (250) disposed between the topsheet and the backsheet, the chassis having a body-facing surface (35, 220A, 520A, 620A, 830A, 930A), an outer-facing surface (231B, 420B, 520B, 620B, 730B, 830B, 930B) on the opposite surface facing away from the wearer's body during use, and the chassis being defined by a periphery (30) comprising first and second longitudinal edges (30A, 30B);
a pair of discrete side flaps (40A, 40B, 440A, 540A, 640A, 740A, 840A, 940A, 1040A, 1040B) each having a first surface (61, 65) and a second surface (63, 67) opposite the first surface and each further comprising attached areas (41, 47, 541A, 641A, 641B, 641C, 841A, 841B, 941A, 941B, 941C) and free ends (43, 49), wherein the attached area of each side flap is attached to the chassis such that a portion of the first surface of each side flap is attached to the outer-facing surface of the chassis, wherein an adhesive is disposed on a portion of the first surface of each of the side flaps adjacent the free end; and
a release liner (275) covering at least a portion of the adhesive on the first surface of each of the side flaps, **characterised in that** the release liner and the free ends of each of the side flaps being disposed vertically superjacent to the chassis when the pair of discrete side flaps is in an unfolded state and oriented where the topsheet is positioned superjacent to the absorbent core.

2. The absorbent article of Claim 1, wherein each side flap comprises a plurality of attached areas configured such that the first surface is attached to the body-facing surface and the outer-facing surface of the chassis.

3. The absorbent article of any of the preceding claims, wherein each of the side flaps further comprises a cuff.

4. The absorbent article of any of the preceding claims, further comprising a pair of flange seams, one flange seam comprising a portion of the first longitudinal edge and the attached area of one of the pair of side flaps, another flange seam comprising a portion of the second longitudinal edge and the attached end of another of the pair of side flaps, wherein each flange seam is disposed laterally outboard of the absorbent core such that the flange seams form application guides.

5. The absorbent article of Claim 1, wherein the outer-facing surface of the chassis comprises an outer-facing surface of the topsheet.

6. The absorbent article of any of the preceding claims, wherein the outer-facing surface of the chassis comprises an outer-facing surface of the backsheet.

7. The absorbent article of any of the preceding claims, further comprising a second pair of side flaps longitudinally spaced from the pair of side flaps.

8. The absorbent article of any of the preceding claims, wherein each of the side flaps is a discrete component attached to the chassis.

9. The absorbent article of any of the preceding claims, wherein the chassis comprises a first longitudinal edge (30A, 1030A) and a second longitudinal edge (30B, 1030B), wherein each of the first and second longitudinal edges are curvilinear and wherein each of the pair of side flaps comprise a side flap end adjacent the attached area, and wherein one of the side flap ends (51, 451, 751) is coterminous with the first longitudinal edge and one of the side flap ends is coterminous with the second longitudinal edge.

10. The absorbent article of any of the preceding claims, wherein each side flap comprises a laminate structure comprising a nonwoven and an elastomeric film.

## Patentansprüche

1. Absorptionsartikel (10, 1000) mit einer Längsachse (81, 1081), einer Querachse (91, 1091), die sich im Allgemeinen lotrecht zu der Längsachse erstreckt, und einer vertikalen Achse (71), die sich im Allgemeinen lotrecht zu der Längsachse und der Querachse erstreckt, wobei der Absorptionsartikel ferner Folgendes umfasst:
eine Grundeinheit (20, 1020), umfassend eine Oberschicht (220, 420, 520, 620), eine Unterschicht (230, 730, 830, 930) und einen Absorptionskern (250), der zwischen der Oberschicht und der Unterschicht angeordnet ist, wobei die Grundeinheit eine körperseitige Oberfläche (35, 220A, 520A, 620A, 830A, 930A), eine körperabgewandte Oberfläche (231B, 420B, 520B, 620B, 730B, 830B, 930B) auf der gegenüberliegenden Oberfläche aufweist, die von dem Körper des Trägers während der Verwendung abgewandt ist, und wobei die Grundeinheit durch einen Umfang (30) definiert wird, umfassend einen ersten und einen zweiten Längsrand (30A, 30B);
ein Paar diskreter Seitenklappen (40A, 40B, 440A, 540A, 640A, 740A, 840A, 940A, 1040A, 1040B), jeweils eine erste Oberfläche (61, 65) und eine der ersten Oberfläche gegenüberliegende zweite Oberfläche (63, 67) aufweisend und ferner jeweils befestigte Bereiche (41, 47, 541A, 641A, 641B, 641C, 841A, 841B, 941A, 941B, 941C) und freie Enden (43, 49) umfassend, wobei der befestigte Bereich auf jeder Seitenklappe an der Grundeinheit befestigt ist, sodass ein Abschnitt der ersten Oberfläche von jeder Seitenklappe an der körperabgewandten Oberfläche der Grundeinheit befestigt ist, wobei ein Klebstoff auf einem Abschnitt der ersten Oberfläche von jeder der dem freien Ende benachbarten Seitenklappen angeordnet ist; und
eine Abziehfolie (275), die wenigstens einen Teil des Klebstoffs auf der ersten Oberfläche von jeder der Seitenklappen abdeckt, **dadurch gekennzeichnet, dass** die Abziehfolie und die freien Enden von jeder der Seitenklappen vertikal die Grundeinheit überlagernd angeordnet sind, wenn das Paar diskreter Seitenklappen in einem ungefalteten Zustand ist und so ausgerichtet ist, dass die Oberschicht den Absorptionskern überlagernd positioniert ist.

2. Absorptionsartikel nach Anspruch 1, wobei jede Seitenklappe eine Vielzahl von befestigten Bereichen aufweist, die so konfiguriert sind, dass die erste Oberfläche an der körperseitigen Oberfläche und der körperabgewandten Oberfläche der Grundeinheit befestigt wird.

3. Absorptionsartikel nach einem der vorstehenden Ansprüche, wobei jede von den Seitenklappen ferner ein Bündchen umfasst.

4. Absorptionsartikel nach einem der vorstehenden Ansprüche, ferner umfassend ein Paar hervorstehender Randnähte, wobei eine hervorstehende Randnaht einen Abschnitt des ersten Längsrands und den befestigten Bereich von einem des Paars von Seitenklappen umfasst, wobei eine andere hervorstehende Randnaht einen Abschnitt des zweiten Längsrands und das befestigte Ende von einem anderen des Paars von Seitenklappen umfasst, wobei jede hervorstehende Randnaht quer nach außen von dem Absorptionskern angeordnet ist, sodass die hervorstehenden Randnähte Anwendungsführungen bilden.

5. Absorptionsartikel nach Anspruch 1, wobei die körperabgewandte Oberfläche der Grundeinheit eine körperabgewandte Oberfläche der Oberschicht aufweist.

6. Absorptionsartikel nach einem der vorstehenden Ansprüche, wobei die körperabgewandte Oberfläche der Grundeinheit eine körperabgewandte Oberfläche der Unterschicht umfasst.

7. Absorptionsartikel nach einem der vorstehenden Ansprüche, ferner umfassend ein zweites Paar von Seitenklappen, die längs von dem Paar von Seitenklappen beabstandet sind.

8. Absorptionsartikel nach einem der vorstehenden Ansprüche, wobei jede von den Seitenklappen ein diskreter Bestandteil ist, der an dem Rahmen befestigt ist.

9. Absorptionsartikel nach einem der vorstehenden Ansprüche, wobei die Grundeinheit einen ersten Längsrand (30A, 1030A) und einen zweiten Längsrand (30B, 1030B) umfasst, wobei jeder von dem ersten und dem zweiten Längsrand krummlinig ist und wobei jedes von dem Paar von Seitenklappen ein dem befestigten Bereich benachbartes Seitenklappenende umfasst, und wobei eines von den Seitenklappenenden (51, 451, 751) an den ersten Längsrand anstößt und eines von den Seitenklappenenden an den zweiten Längsrand anstößt.

10. Absorptionsartikel nach einem der vorstehenden Ansprüche, wobei jede Seitenklappe eine Laminatstruktur umfasst, die ein Vlies und eine Elastomerfolie umfasst.

## Revendications

1. Article absorbant (10, 1000) ayant un axe longitudinal (81, 1081), un axe latéral (91, 1091) s'étendant généralement perpendiculairement à l'axe longitudinal, et un axe vertical (71) s'étendant généralement perpendiculairement à l'axe longitudinal et à l'axe latéral, l'article absorbant comprenant en outre :
un châssis (20, 1020) comprenant une feuille de dessus (220, 420, 520, 620), une feuille de fond (230, 730, 830, 930), et une âme absorbante (250) disposée entre la feuille de dessus et la feuille de fond, le châssis ayant une surface faisant face au corps (35, 220A, 520A, 620A, 830A, 930A), une surface tournée vers l'extérieur (231B, 420B, 520B, 620B, 730B, 830B, 930B) sur la surface opposée opposée au corps du porteur durant l'utilisation, et le châssis étant défini par une périphérie (30) comprenant des premier et deuxième bords longitudinaux (30A, 30B) ;
une paire de rabats latéraux distincts (40A, 40B, 440A, 540A, 640A, 740A, 840A, 940A, 1040A, 1040B) ayant chacun une première surface (61, 65) et une deuxième surface (63, 67) opposée à la première surface et chacun comprenant en outre des zones fixées (41, 47, 541A, 641A, 641B, 641C, 841A, 841B, 941A, 941B, 941C) et des extrémités libres (43, 49), dans lequel la zone fixée de chaque rabat latéral est fixée au châssis de telle sorte qu'une partie de la première surface de chaque rabat latéral est fixée à la surface tournée vers l'extérieur du châssis, dans lequel un adhésif est disposé sur une partie de la première surface de chacun des rabats latéraux adjacents à l'extrémité libre ; et
une protection détachable (275) recouvrant au moins une partie de l'adhésif sur la première surface de chacun des rabats latéraux, **caractérisé en ce que** la protection détachable et les extrémités libres de chacun des rabats latéraux sont disposées verticalement de manière surjacente au châssis lorsque la paire de rabats latéraux distincts est dans un état déplié et orienté là où la feuille de dessus est positionnée de manière surjacente à l'âme absorbante.

2. Article absorbant selon la revendication 1, dans lequel chaque rabat latéral comprend une pluralité de zones fixées configurées de telle sorte que la première surface est fixée à la surface tournée vers le corps et à la surface tournée vers l'extérieur du châssis.

3. Article absorbant selon l'une quelconque des revendications précédentes, dans lequel chacun des rabats latéraux comprend en outre un revers.

4. Article absorbant selon l'une quelconque des revendications précédentes, comprenant en outre une paire de coutures de bride, une couture de bride comprenant une partie du premier bord longitudinal et de la zone fixée d'un de la paire de rabats latéraux, une autre couture de bride comprenant une partie du deuxième bord longitudinal et de l'extrémité fixée d'un autre de la paire de rabats latéraux, dans lequel chaque couture de bride est disposée latéralement à l'extérieur de l'âme absorbante de telle sorte que les coutures de bride forment des guides d'application.

5. Article absorbant selon la revendication 1, dans lequel la surface tournée vers l'extérieur du châssis comprend une surface tournée vers l'extérieur de la feuille de dessus.

6. Article absorbant selon l'une quelconque des revendications précédentes, dans lequel la surface tournée vers l'extérieur du châssis comprend une surface tournée vers l'extérieur de la feuille de fond.

7. Article absorbant selon l'une quelconque des revendications précédentes, comprenant en outre une deuxième paire de rabats latéraux espacés longitudinalement de la paire de rabats latéraux.

8. Article absorbant selon l'une quelconque des revendications précédentes, dans lequel chacun des rabats latéraux est un composant distinct fixé au châssis.

9. Article absorbant selon l'une quelconque des revendications précédentes, dans lequel le châssis comprend un premier bord longitudinal (30A, 1030A) et un deuxième bord longitudinal (30B, 1030B), dans lequel chacun des premier et deuxième bords longitudinaux est curviligne et dans lequel chacun de la paire de rabats latéraux comprend une extrémité de rabat latéral adjacente à la zone fixée, et dans lequel l'une des extrémités de rabat latéral (51, 451, 751) est contiguë avec le premier bord longitudinal et l'une des extrémités de rabat latéral est contiguë avec le deuxième bord longitudinal.

10. Article absorbant selon l'une quelconque des revendications précédentes, dans lequel chaque rabat latéral comprend une structure stratifiée comprenant un nontissé et un film élastomère.
